Europäisches Patentamt

European Patent Office

Office européen des brevets

(19)

(11) Veröffentlichungsnummer: **0 301 368 B1**

(12) **EUROPÄISCHE PATENTSCHRIFT**

(45) Veröffentlichungstag der Patentschrift: **27.11.91**

(51) Int. Cl.⁵: **A61F 5/01**

(21) Anmeldenummer: **88111568.7**

(22) Anmeldetag: **19.07.88**

(54) **Cervicalstütze.**

(30) Priorität: **28.07.87 DE 3724885**

(43) Veröffentlichungstag der Anmeldung:
**01.02.89 Patentblatt 89/05**

(45) Bekanntmachung des Hinweises auf die
Patenterteilung:
**27.11.91 Patentblatt 91/48**

(84) Benannte Vertragsstaaten:
**AT BE CH DE ES FR GB GR IT LI LU NL SE**

(56) Entgegenhaltungen:
**CA-A- 1 067 371      GB-A- 1 132 607**
**US-A- 2 474 200      US-A- 2 904 040**
**US-A- 3 024 784      US-A- 3 364 926**
**US-A- 3 512 523**

(73) Patentinhaber: **Heimann, Dieter, Dr.**
**Königsberger Ring 99**
**W-2340 Kappeln(DE)**

(72) Erfinder: **Heimann, Dieter, Dr.**
**Königsberger Ring 99**
**W-2340 Kappeln(DE)**

(74) Vertreter: **Wehser, Wulf, Dipl.-Ing. et al**
**Patentanwälte Wehser & Fleuchaus Ro-**
**scherstrasse 12**
**W-3000 Hannover 1(DE)**

## Beschreibung

Die Erfindung betrifft eine Cervicalstütze zum Ruhigstellen der Halswirbelsäule in Form einer den Hals des Patienten umfassenden gepolsterten Manschette.

Bekannte Cervicalstützen dieser Art (US-A-4,099,523) bestehen aus zwei gegeneinander verschieblichen starren Abschnitten, deren vertikaler Abstand einstellbar ist, um einerseits die Cervicalstütze an verschiedene Körperformen anzupassen und andererseits zur Entlastung der Halswirbelsäule einen gewissen Druck auf die zu stützenden Partien des Kopfes auszuüben.

Nachteilig hierbei ist es, daß durch die bekannten Cervicalstützen der Kopf des Patienten insgesamt unbeweglich gehalten wird, was zu einem unangenehmen Druckgefühl, zu Verspannungen der Muskeln und - insbesondere bei längerem Tragen - auch zu deren Schwächung führen kann.

Der Erfindung liegt demgemäß die Aufgabe zugrunde, eine Cervicalstütze der eingangs genannten Art so auszubilden, daß trotz der Ruhigstellung in dem hierfür notwendigen Bereich eine gewisse Beweglichkeit des Kopfes zur Betätigung der Muskeln und zur Verhinderung eines auf die Dauer unangenehmen Druckgefühles möglich ist.

Diese Aufgabe wird erfindungsgemäß dadurch gelöst, daß die Cervicalstütze in einem zu stützenden Bereich des Kopfes, zum Verhindern schmerzhafter Bewegungen, relativ starr und in einem weiteren Bereich, zum Ermöglichen einer gewissen Bewegungsfreiheit in der schmerzfreien Richtung, elastisch verformbar ausgebildet ist.

Mit dieser Anordnung wird erreicht, daß der Stützbereich, also der immobilisierende Bereich, schmerzhafte Bewegungen verhindert, während der elastisch verformbare Bereich dem Patienten eine hinreichende Bewegungsfreiheit in der schmerzfreien Richtung, also auf der dem Stützbereich abgewandten Seite ermöglicht.

Mit der erfindungsgemäßen Cervicalstütze wird also die Halswirbelsäule nur dort ruhiggestellt, wo es unbedingt notwendig ist. Ansonsten bleibt die freie Beweglichkeit des Kopfes erhalten.

In Abhängigkeit von den jeweiligen Erfordernissen kann zweckmäßigerweise der Stützbereich an jeder Stelle des Halses angebracht sein. Der Stützbereich kann hierbei einen Umfangsabschnitt von etwa 90° umfassen, wobei der übrige Bereich beweglich gehalten werden kann.

In der Regel dürfte der Stützbereich auf einer Seite der Medianachse des Halses liegen, wobei er auch einen Umfangsabschnitt größer als 90° umfassen kann, während der verformbare Bereich dem Stützbereich gegenüberliegend angeordnet ist.

In Ausnahmefällen sind aber auch andere Anordnungen möglich, beispielsweise eine Ruhigstellung im Nacken und eine Beweglichkeit im Kinnbereich oder umgekehrt.

Zur wahlweisen Festlegung des starren bzw. des nachgiebigen Bereiches sind zweckmäßigerweise zwei im vertikalen Abstand zueinander angeordnete Rahmenabschnitte vorgesehen, die im Tragezustand der Stütze etwa horizontal verlaufen und übereinander angeordnet sind und zwischen denen wenigstens zwei Abstandshalter angeordnet sind, die in verschiedenen Abstandslagen feststellbar sind.

Auf diese Weise wird erreicht, daß im Bereich des nicht festgestellten Abstandshalters die beiden Rahmenabschnitte gegeneinander beweglich sind, während sie im Bereich eines festgestellten Abstandshalters starr miteinander verbunden sind. Auf diese Weise lassen sich wahlweise die Stützbereiche und die Bewegungsbereiche festlegen. Insbesondere sind diese Bereiche gegeneinander vertauschbar.

Besonders zweckmäßig ist es, wenn die Abstandshalter als Druckfedern ausgebildet sind oder solche enthalten, wobei bei nicht festgestelltem Abstandshalter eine Bewegung des Kopfes gegen die Federkraft möglich ist.

Die Rahmenabschnitte können ebenfalls aus einem elastischen Material bestehen, beispielsweise aus einem elastisch verformbaren Kunststoff, so daß sie nach einer Verformung in ihre Ausgangslage zurückkehren.

Der obere Rahmenabschnitt und der untere Rahmenabschnitt können im Bereich ihrer Enden jeweils mit einem aus demselben Material bestehenden Bogen miteinander verbunden sein.

Die Abstandshalter sind zweckmäßigerweise innerhalb einer sich über den größten Teil des Halsumfanges erstreckenden Ausnehmung der Cervicalstütze angeordnet, die oben und unten durch die beiden Rahmenabschnitte begrenzt ist.

Die Abstandshalter können als teleskopartig gegeneinander verschiebbare Laschen ausgebildet sein, von denen eine oder beide jeweils mit einem Langloch versehen sind, welches von einer Feststellschraube durchgriffen wird.

Anstelle der Laschen können auch zwei teleskopartig ineinander eingreifende Rohrstücke vorgesehen sein, die ebenfalls in bestimmten Einstellagen gegeneinander feststellbar sind, wobei innerhalb des äußeren Rohrstückes eine Druckfeder angeordnet sein kann, die sich im äußeren Rohrstück abstützt und deren freies Ende das innere Rohrstück beaufschlagt.

Das innere Rohrstück kann mit im Abstand zueinander angeordneten Ringnuten versehen sein, die der Aufnahme eines O-Ringes dienen, um die beiden Rohrstücke gegeneinander festzusetzen.

Um den Eintritt des O-Ringes in die Ausnehmung des äußeren Rohrstückes zu erleichtern, ist die dem inneren Rohrstück zugewandte Öffnung des äußeren Rohrstückes konisch ausgebildet.

Die Erfindung wird im folgenden anhand von Ausführungsbeispielen in der Zeichnung näher erläutert.

Fig. 1 zeigt in perspektivischer Darstellung eine Cervicalstütze, auf welche die Erfindung Anwendung findet.

Fig. 2 zeigt in perspektivischer Darstellung eine abgewandelte Ausführungsform.

Fig. 3 zeigt im Schnitt eine weitere Ausführungsform der Abstandshalter.

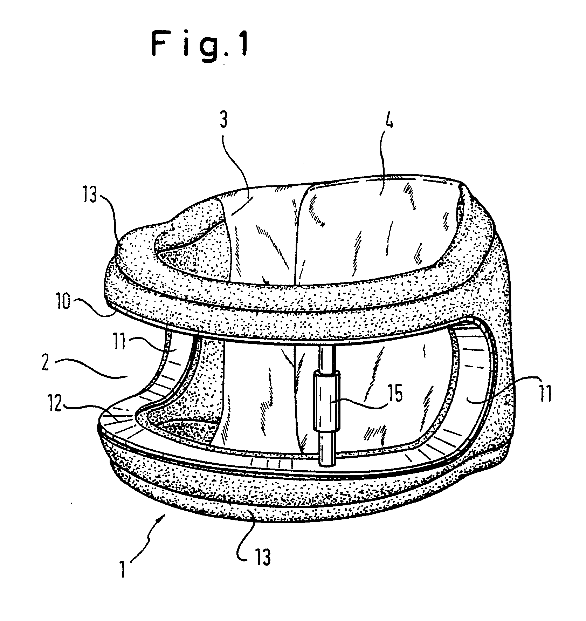

Gemäß Fig. 1 weist die Cervicalstütze 1 eine Ausnehmung 2 in ihrem im Tragezustand dem vorderen Halsbereich des Trägers zugewandten Bereich mit einem oberen Rahmenabschnitt 10 und einem unteren Rahmenabschnitt 12 auf, die beide im Bereich ihrer Enden durch jeweils einen Bogen 11 miteinander verbunden sind. Die Rahmenabschnitte 10 und 12 sowie der Verbindungsbogen sind flexibel ausgebildet, so daß sich die Ausnehmung 2 zusammendrücken läßt.

Auf ihrer Ober- bzw. Unterseite sind die Rahmenabschnitte 10 und 12 mit weichen Polstern 13, vorzugsweise aus Schaumstoff versehen.

Gemäß Fig. 1 ist ein federnd ausgebildeter und in seiner Länge einstellbarer Abstandshalter 15 vorgesehen, der an den oberen und unteren Rahmenabschnitten 10 und 12 angreift. Im Bereich des Abstandshalters 15 kann also die Cervicalstütze nicht zusammengedrückt werden, während in den übrigen Bereichen ein Zusammendrücken der Ausnehmung 2 möglich ist, so daß in diesen Bereichen eine Bewegung des Kopfes erhalten bleibt.

Bei der Ausführungsform nach Fig. 1 erstreckt sich die Ausnehmung 2 über einen Umfang von wenigstens 180° der Cervicalstütze in deren geschlossenen Zustand.

An den Ausnehmungsbereich der Cervicalstütze schließen zwei Verschlußlaschen 3 und 4 an, die auf ihren einander zugewandten Flächen jeweils mit einem Abschnitt eines Klettverschlusses versehen sind.

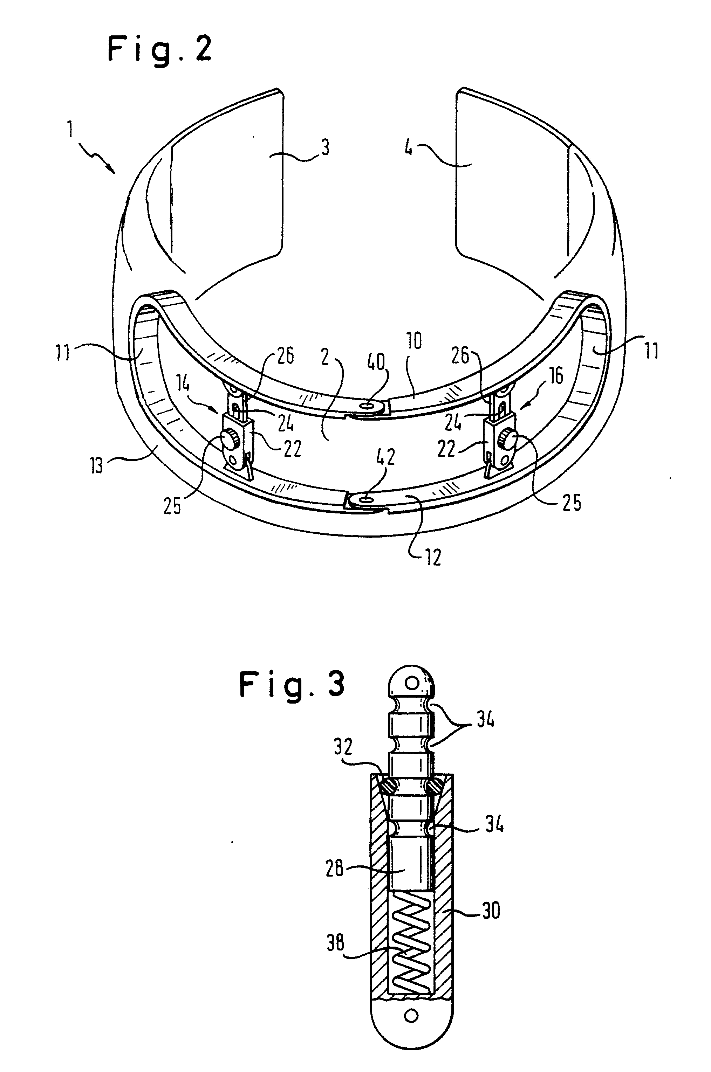

Fig. 2 zeigt eine etwas abgewandelte Ausführungsform der Cervicalstütze. Es sind dort zwei Abstandshalter 14 und 16 vorgesehen, die ebenfalls mit ihren Enden an den beiden Rahmenabschnitten 10 und 12 angreifen. Die Abstandshalter sind jeweils mit teleskopartig gegeneinander verschiebbaren Laschen 22 und 26 versehen, von denen eine oder beide jeweils ein Langloch 24 aufweisen, welches von einer Feststellschraube 25 durchgriffen wird. Bei gelöster Feststellschraube 25 kann mithin der Abstand zwischen den beiden Rahmenabschnitten 10 und 12 eingestellt werden.

Bei einem Zusammendrücken der Ausnehmung 2 im beweglichen Bereich kann die elastische Verformbarkeit der Bögen 11 sowie der Rahmenabschnitte 10 und 12 ausreichen, um den Kopf des Trägers in Ruhelage leicht gestützt zu halten, andererseits aber die Bewegung des Kopfes im Verformungsbereich zu ermöglichen.

In Fig. 2 sind noch zwei Gelenke 40 und 42 angedeutet, die ein Aufklappen der Cervicalstütze ermöglichen, um diese leichter um den Hals des Trägers legen zu können.

Fig. 3 zeigt eine abgewandelte Ausführungsform eines Abstandshalters, wobei hier der Abstandshalter aus zwei teleskopartig ineinander eingreifenden Rohrstücken 28 und 30 besteht. Innerhalb des äußeren Rohrstückes 30 ist eine Druckfeder 38 angeordnet, die sich im äußeren Rohrstück 30 abstützt und deren freies Ende das innere Rohrstück 28 beaufschlagt.

Das innere Rohrstück 28 ist mit mehreren im Abstand zueinander angeordneten Ringnuten 34 versehen, die der Aufnahme eines O-Ringes dienen, um die beiden Rohrstücke gegeneinander festzusetzen. Wird der O-Ring in eine der oberen Ringkerben 34 eingelegt, kann das innere Rohrstück 28 gegen die Kraft der Druckfeder 38 vergleichsweise leicht in das äußere Rohrstück 30 hineingedrückt werden. Beim Einsatz des O-Ringes 32 in eine der unteren Ringnuten wird das innere Rohrstück gegen das äußere Rohrstück verspannt.

Um den Eintritt des O-Ringes 32 in die Ausnehmung des äußeren Rohrstückes 30 zu erleichtern, ist die dem äußeren Rohrstück 28 zugewandte Öffnung konisch ausgebildet.

**Patentansprüche**

1. Cervicalstütze zum Ruhigstellen der Halswirbelsäule in Form einer den Hals des Patienten umfassenden gepolsterten Manschette, dadurch gekennzeichnet, daß die Cervicalstütze (1) in einem zu stützenden Bereich des Kopfes, zum Verhindern schmerzhafter Bewegungen, relativ starr und in einem weiteren Bereich, zum Ermöglichen einer gewissen Bewegungsfreiheit in der schmerzfreien Richtung, elastisch verformbar ausgebildet ist.

2. Cervicalstütze nach Anspruch 1, dadurch gekennzeichnet, daß der Stützbereich einen Umfangsabschnitt von etwa 90° umfaßt, wobei der übrige Bereich verformbar ist.

3. Cervicalstütze nach Anspruch 1, dadurch gekennzeichnet, daß der Stützbereich einen Umfangsabschnitt größer als 90° umfaßt und daß der verformbare Bereich dem Stützbereich gegenüberliegt.

4. Cervicalstütze nach Anspruch 2 und 3, dadurch gekennzeichnet, daß der Stützbereich auf einer Seite der Medianachse des Halses liegt.

5. Cervicalstütze nach Anspruch 2 oder 3, dadurch gekennzeichnet, daß der Stützbereich im Nacken und der verformbare Bereich im Kinnbereich liegt oder umgekehrt.

6. Cervicalstütze nach einem der vorhergehenden Ansprüche, dadurch gekennzeichnet, daß zwei im vertikalen Abstand zueinander angeordnete Rahmenabschnitte (10,12) vorgesehen sind, die im Tragezustand der Stütze (1) etwa horizontal verlaufen und übereinander angeordnet sind und zwischen denen wenigstens zwei Abstandshalter (14,15,16) angeordnet sind, die in verschiedenen Abstandslagen feststellbar sind.

7. Cervicalstütze nach Anspruch 6, dadurch gekennzeichnet, daß der Stützbereich gegen den verformbaren Bereich durch entsprechendes Lösen bzw. Festsetzen der Abstandshalter vertauschbar ist.

8. Cervicalstütze nach Anspruch 6 oder 7, dadurch gekennzeichnet, daß die Abstandshalter (14,15,16) als Druckfedern ausgebildet sind oder solche enthalten, wobei bei nicht festgestelltem Abstandshalter eine Bewegung des Kopfes gegen die Federkraft möglich ist.

9. Cervicalstütze nach einem der Ansprüche 6 bis 8, dadurch gekennzeichnet, daß die Rahmenabschnitte (10,12) aus einem elastischen Material, vorzugsweise aus einem elastisch verformbaren Kunststoff bestehen, so daß sie nach einer Verformung in ihre Ausgangslage zurückkehren.

10. Cervicalstütze nach Anspruch 9, dadurch gekennzeichnet, daß die beiden Rahmenabschnitte (10,12) im Bereich ihrer Enden jeweils mit einem aus demselben Material bestehenden Bogen (11) miteinander verbunden sind.

11. Cervicalstütze nach einem der Ansprüche 6 bis 10, dadurch gekennzeichnet, daß die Abstandshalter (14,15,16) innerhalb einer sich über den größten Teil des Halsumfanges erstreckenden Ausnehmung (2) der Cervicalstütze (1) angeordnet sind, die oben und unten durch die beiden Rahmenabschnitte (10,12) begrenzt ist.

12. Cervicalstütze nach einem der Ansprüche 6 bis 11, dadurch gekennzeichnet, daß die Abstandshalter (14,16) als teleskopartig ineinander verschiebbare Laschen (22,26) ausgebildet sind, von denen eine oder beide jeweils mit einem Langloch (24) versehen sind, welches von einer Feststellschraube (25) durchgriffen wird.

13. Cervicalstütze nach einem der Ansprüche 6 bis 11, dadurch gekennzeichnet, daß die Abstandshalter als zwei teleskopartig ineinander eingreifende Rohrstücke (28,30) ausgebildet sind, die in bestimmten Einstellagen gegeneinander feststellbar sind.

14. Cervicalstütze nach Anspruch 13, dadurch gekennzeichnet, daß innerhalb des äußeren Rohrstückes (30) eine Druckfeder (38) angeordnet ist, die sich im äußeren Rohrstück (30) abstützt und deren freies Ende das innere Rohrstück (28) beaufschlagt.

15. Cervicalstütze nach Anspruch 14, dadurch gekennzeichnet, daß das innere Rohrstück (28) mit im Abstand zueinander angeordneten Ringnuten (34) versehen ist, die der Aufnahme eines O-Ringes (32) dienen, um die beiden Rohrstücke (28,30) gegeneinander festzusetzen.

16. Cervicalstütze nach Anspruch 15, dadurch gekennzeichnet, daß die dem inneren Rohrstück (28) zugewandte Öffnung des äußeren Rohrstückes (30) konisch ausgebildet ist.

## Claims

1. Cervical support for holding the cervical vertebral column in the form of a padded collar which surrounds the neck of the patient, characterised in that the cervical support (1) is constructed to be relatively rigid in an area of the head (1) which is to be supported in order to prevent painful movements, and elastically deformable in another area (2) in order to permit a certain amount of freedom of movement in the pain-free direction.

2. Cervical support according to claim 1, characterised in that the support area includes a circumferential portion of approximately 90°, and that the remaining area is deformable.

3. Cervical support according to claim 1, characterised in that the support area includes a circumferential portion of more than 90°, and that the deformable area is located opposite the support area.

4. Cervical support according to claim 2 and 3, characterised in that the support area is located on one side of the median axis of the neck.

5. Cervical support according to claim 2 or 3, characterised in that the support area is located in the neck region and the deformable area in the chin region, or vice versa.

6. Cervical support according to one of the above claims, characterised in that two frame portions (10, 12) are provided, which are arranged at a vertical distance from one another and which, when the support (1) is worn, extend approximately horizontally and are arranged on top of one another, and between which are at least two spacers (14, 15, 16), which can be fixed in differently spaced positions.

7. Cervical support according to claim 6, characterised in that the support area is interchangeable with the deformable area by releasing or fixing of the spacers respectively.

8. Cervical support according to claim 6 or 7, characterised in that the spacers (14, 15, 16) are constructed to be pressure springs or to contain such springs, in which respect a head movement against the spring force is possible if the spacers are not fixed.

9. Cervical support according to one of claims 6 to 8, characterised in that the frame portions (10, 12) are made of an elastic material, preferably of an elastically deformable synthetic material, so that they return into their initial position after having been deformed.

10. Cervical support according to claim 9, characterised in that the two frame portions (10, 12) are connected to one another in their end areas by a bend (11), which is made of the same material.

11. Cervical support according to one of the claims 6 to 10, characterised in that the spacers (14, 15, 16) are arranged within an aperture (2) of the cervical support (1), which aperture extends over the major portion of the neck circumference and is defined at the top and bottom by both frame portions (10, 12).

12. Cervical support according to one of claims 6 to 11, characterised in that the spacers (14, 16) are constructed to be telescopically retractable tongues (22, 26) one or both of which being provided with an oblong hole (24) which is passed through by a securing bolt (25).

13. Cervical support according to one of the claims 6 to 11, characterised in that the spacers are constructed to be two telescopically inter-engaging tubular portions (28, 30), which are secured against one another in chosen setting positions.

14. Cervical support according to claim 13, characterised in that a pressure spring (38) is arranged within the outer tubular portion (30), which spring supports itself in the outer tubular portion (30), and the free end of which loads the inner tubular portion (28).

15. Cervical support according to claim 14, characterised in that the inner tubular portion (28) is provided with annular grooves (34) at a distance from each other, which grooves serve to receive an O-ring (32) for fixing both tubular portions (28, 30) relative to one another.

16. Cervical support according to claim 15, characterised in that the aperture, which faces towards the inner tubular portion (28), of the outer tubular portion (30) is arranged to be conical.

**Revendications**

1. Soutien cervical pour immobiliser la colonne vertébrale cervicale se présentant sous la forme d'un manchon rembourré entourant le cou du patient, caractérisé en ce que le soutien cervical (1) est conçu, dans une région soutenant la tête, de manière relativement rigide pour empêcher des mouvements douloureux et dans une autre région, de manière élastiquement déformable, pour permettre une certaine liberté de mouvement dans la direction indolore.

2. Soutien cervical selon la revendication 1, caractérisé en ce que la zone de soutien comprend une partie de circonférence d'environ 90°, le reste étant déformable.

3. Soutien cervical selon la revendication 1, caractérisé en ce que la zone de soutien comprend une partie de circonférence supérieure à 90° et en ce que la zone déformable fait face à la zone de soutien.

4. Soutien cervical selon les revendications 2 et 3, caractérisé en ce que la zone de soutien se situe sur un côté de l'axe médian du cou.

5. Soutien cervical selon la revendication 2 ou 3, caractérisé en ce que la zone de soutien se situe dans la nuque et la zone déformable dans la région du menton ou inversement.

6. Soutien cervical selon l'une des revendications précédentes, caractérisé en ce qu'il est prévu deux parties de cadre (10, 12) espacées verticalement l'une de l'autre, qui à l'état porté du soutien (1), s'étendent à peu près horizontalement et sont superposées et entre lesquelles sont placés au moins deux écarteurs (14, 15, 16) qui peuvent être bloqués dans différentes positions d'écartement.

7. Soutien cervical selon la revendication 6, caractérisé en ce que la zone de soutien peut être échangée contre la zone déformable, par détachement ou blocage correspondant des écarteurs.

8. Soutien cervical selon la revendication 6 ou 7, caractérisé en ce que les écarteurs (14, 15, 16) sont ou contiennent des ressorts de pression, un mouvement de la tête à l'encontre de la force de ressort étant possible, lorsque les écarteurs ne sont pas bloqués.

9. Soutien cervical selon l'une des revendications 6 à 8, caractérisé en ce que les parties de cadre (10, 12) sont réalisées dans un matériau élastique, de préférence dans une matière plastique élastiquement déformable, ce qui fait qu'elles se replacent dans leur position initiale, après une déformation.

10. Soutien cervical selon la revendication 9, caractérisé en ce que les deux parties de cadre (10, 12) sont assemblées entre elles dans la région de leurs extrémités, chacune avec un arc (11) réalisé dans le même matériau.

11. Soutien cervical selon l'une des revendications 6 à 10, caractérisé en ce que les écarteurs (14, 15, 16) sont placés à l'intérieur d'un évidement (2) du soutien cervical (1) s'étendant sur la plus grande partie de la circonférence du cou, l'évidement étant limité en haut et en bas par les deux parties de cadre (10, 12).

12. Soutien cervical selon l'une des revendication 6 à 11, caractérisé en ce que les écarteurs (14, 16) sont des éclisses (22, 26) coulissant l'une dans l'autre de manière télescopique, dont une ou les deux sont pourvues chacune d'un trou allongé (24) qui est traversé par une vis de blocage (25).

13. Soutien cervical selon l'une des revendications 6 à 11, caractérisé en ce que les écarteurs sont des pièces de tube (28, 30) s'engageant l'une dans l'autre de manière télescopique, qui peuvent être bloquées l'une par rapport à l'autre dans des positions de réglage déterminées.

14. Soutien cervical selon la revendication 13, caractérisé en ce qu'à l'intérieur de la pièce de tube (30) extérieure, est placé un ressort de pression (38) qui prend appui dans la pièce de tube extérieure (30) et dont l'extrémité libre agit sur la pièce de tube intérieure (28).

15. Soutien cervical selon la revendication 14, caractérisé en ce que la pièce de tube intérieure (28) est pourvue de rainures annulaires (34) espacées l'une de l'autre, qui servent à loger un joint torique (32), afin de bloquer les deux pièces de tube (28, 30) l'une par rapport à l'autre.

16. Soutien cervical selon la revendication 15, caractérisé en ce que l'ouverture de la pièce de tube extérieure (30), tournée vers la pièce de tube intérieure (28), est conique.

# F i g.1

# Fig. 2

# Fig. 3